# EUROPEAN PATENT APPLICATION

(11) **EP 4 215 179 A1**
(43) Date of publication of application: **26.07.2023**
(21) Application number: 22152754.2
(22) Date of filing: 21.01.2022
(51) Int. Cl.: A61K 9/00, A61K 9/107, A61L 26/00

(54) **HYDROGEL COMPRISING DENDRITIC POLYGLYCEROL UNITS AND POLYETHER UNITS**

(71) Applicant: Freie Universität Berlin, 14195 Berlin (DE)
(72) Inventor: HAAG, Rainer, 12209 Berlin (DE); THONGROM, Boonya, 13403 Berlin (DE)
(74) Representative: Maikowski & Ninnemann Patentanwälte Partnerschaft mbB

(57) **Abstract**

The present invention relates to a hydrogel comprising dendritic polyglycerol units and polyether units. The dendritic polyglycerol units correspond to general formula (I). They comprise a dendritic polyglycerol core having terminal hydroxyl groups and terminal sulfate or sulfonate substituents, wherein a degree of substitution of the dendritic polyglycerol core with sulfate or sulfonate substituents lies in a range from 10 % to 98 %. The polyether units correspond to general formula (II), wherein the dendritic polyglycerol units and the polyether units are covalently bound to each other via the bonds illustrated by dashed lines in formula (I) and formula (II). The hydrogel has antiviral properties and can be used, e.g., as mucus replacement, cartilage replacement, or synovial fluid replacement.

## Description

The present invention relates to a hydrogel according to the preamble of claim 1, to the in vitro use of such hydrogel according to the preambles of claims 6 and 8, to the medical uses of such a hydrogel according to claims 9 and 10, to a pharmaceutical composition comprising such a hydrogel according to the preamble of claim 11, as well as to a method for manufacturing such a hydrogel according to the preamble of claim 12.

HSV-1 is a part of the Herpesviridae family, which is known to target oral, pharyngeal and genitals. [1] The virus is supported by a wide range of hosts, including humans. HSV-1 has a high seroprevalence of approximately 80 % in adults.[5] It is an enveloped virus around 155-240 nm in diameter.[1, 2] HSV-1 can be a cause of morbidity and mortality in newborns and immunocompromised patients, and antivirals such as acyclovir (ACV) and ganciclovir are often prescribed to reduce the frequency, duration, and severity of infection.[3, 4]

HSV-1 uses its surface glycoprotein to bind to heparan sulfate proteoglycans (HSPG) to start the infection in host cells. Because the virus entry is primarily driven by electrostatic interactions, many drug designs have been inspired by the sulfate-dominant interactions.[5] This deals with the problem on the long-term scale, disabling the formation of a virus-reservoir. Previously, Dey et. al. fabricated sulfated dendritic polyglycerol (dPGS) nanogels as synthetic heparan sulfate mimics of a range of flexibilities as well as sizes.[6] While all the nanogels were successful in inhibiting virus, the more flexible nanogels showed higher efficacy.

Recently, the inventors' group has also published a new series of dendronized linear polysulfates as multivalent inhibitors to prevent HSV entry. However, in this case, highly flexible sulfated linear polyglycerol (IPGS) was compared with more rigid sulfated architectures. Here also, the flexible IPGS was highly successful in intervening with the infection process, showing 295 times greater efficacy than heparin.[7] Recently, hydrogels are emerging as important platforms for multiple applications in the biomedical arena.[8, 9] Hydrogels are formed when macromolecules crosslink by physical or chemical means to form water-swollen networks. They have a number of characteristics which make them particularly attractive for biomedical applications. For example, due to their structural similarity to living tissues, they are highly biocompatible; moreover they are able to provide low interfacial tension with the nearby tissues in the body.[10] They can also be noninvasively injected and fill in any required shape at the injection point.[11] In addition to other biomedical applications, they have been used in virus-trapping and containment.

Zhang et. al. used glycosylated hydrogels to trap the Norovirus.[12] Importantly, mucus hydrogels within the body function in a similar way providing the first line of defense against pathogenic attack.[13] The role of scaffold flexibility for virus binding is very important, yet, macroscopic materials with different stiffness have not been studied for this application.

WO 2018/041784 A1 describes a combined preparation of a cross-linker by albumin and a polymer comprising at least one albumin-crosslinking group. This combined preparation can be used for treating a cartilage defect.

Baldwin and Kiick [20] describe a reaction between a maleimide-functionalized low molecular weight heparin with various thiol functionalized poly(ethylene glycol) multi-arm star polymers as well as degradation reactions of the resulting product.

It is an object of the present invention to provide a novel antiviral material for various medical applications that has a high biocompatibility and can be easier manufactured than antiviral materials known from prior art.

This object is achieved with a hydrogel having the features of claim 1. Such a hydrogel comprises dendritic polyglycerol units and polyether units. The dendritic polyglycerol units correspond to general formula (I) and the polyether units correspond to general formula (II). In addition, the dendritic polyglycerol units and the polyether units are covalently bound to each other via the bonds illustrated by dashed lines in formula (I) and formula (II):

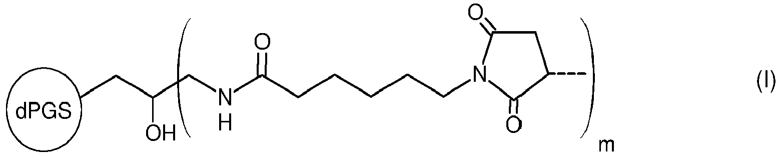

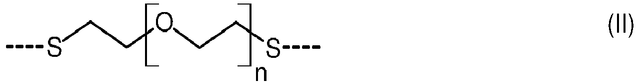

In this context, the abbreviations have the following meaning:
dPGS is a dendritic polyglycerol core comprising terminal hydroxyl groups and terminal sulfate or sulfonate substituents, wherein a degree of substitution of the dendritic polyglycerol core with sulfate or sulfonate substituents lies in a range from 10 % to 98%,
m is 2 % to 10 %, in particular from 3 % to 9 %, in particular from 4 % to 8 %, in particular from 5 % to 7 %, of all terminal groups of the dendritic polyglycerol core that are not substituted with the sulfate or sulfonate substituents,
n is a number lying in range from 1 to 1000, in particular from 2 to 900, in particular from 3 to 800, in particular from 4 to 700, in particular from 5 to 600, in particular from 6 to 500, in particular from 7 to 400, in particular from 8 to 300, in particular from 9 to 200, in particular from 10 to 100, in particular from 20 to 90, in particular from 30 to 80, in particular from 40 to 60.

Thus, the inventors have designed sulfated hydrogels by a click conjugation approach. They have employed polyethylene glycol (PEG) dithiol and (dendritic polyglycerol sulfate) dPGS-maleimide as the macromolecular components to linear and crosslinking units, respectively. This ball-and-chain type system forms a hydrogel as a Michael-click reaction occurs between the linear PEG-dithiol and the maleimides decorating dPGS. These new sulfated hydrogels were studied by rheology and can prevent viral infection to different extents depending on the scaffold flexibility. The large surface area and highly negatively charged system allow virus binding by multivalent polyelectrolyte interactions.

In an embodiment, the degree of substitution of the dendritic polyglycerol core (sometimes also referred to as backbone) lies in a range from 11 % to 97 %, in particular from 12 % to 96 %, in particular from 15 % to 95 %, in particular from 16 % to 94 %, in particular from 17 % to 93 %, in particular from 18 % to 92 %, in particular from 19 % to 91 %, in particular from 20 % to 90 %, in particular from 30 % to 85 %, in particular from 40 % to 70 %, in particular from 50 % to 60 %, in particular from 70 % to 98 %, in particular from 75 % to 95 %, in particular from 80 % to 90 %.

In an embodiment, each dendritic polyglycerol core carries 4 to 13, in particular 5 to 12, in particular 6 to 11, in particular 7 to 10, in particular 8 to 9 maleimide substituents corresponding to general formula (III):

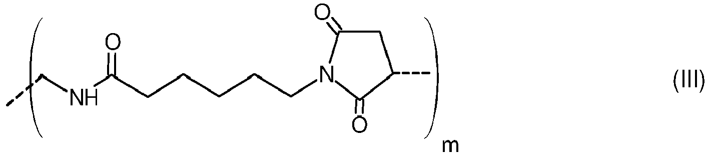

In an embodiment, the hydrogel has a gel concentration lying in a range of from 3 % to 10 %, in particular from 4 % to 9 %, in particular from 5 % to 8 %, in particular from 6 % to 7 %. All of these percentages are to be understood as mass percent (weight per volume; w/v).

In an embodiment, the hydrogel has a mesh size, measured at 37 °C in a non-swollen state of the hydrogel, lying in a range of from 10 nm to 100 nm, in particular from 20 nm to 90 nm, in particular from 30 nm to 80 nm, in particular from 40 nm to 70 nm, in particular from 50 nm to 60 nm. In this context, the mesh size is measured by applying a rheological model which is represented by the following equation: G' = *kT*/*ξ*³*,* wherein k is the Boltzmann constant, T is the temperature and *ξ* is the mesh size. G' is the plateau modulus which is determined at 0.3 Hz.

In an embodiment, the hydrogel has a storage modulus, measured at 37 _{°}C in a non-swollen state of the hydrogel, lying in a range of from 5 Pa to 1200 Pa, in particular from 10 Pa to 1100 Pa, in particular from 20 Pa to 1000 Pa, in particular from 30 Pa to 900 Pa, in particular from 40 Pa to 800 Pa, in particular from 50 Pa to 700 Pa, in particular from 60 Pa to 600 Pa, in particular from 70 Pa to 500 Pa, in particular from 80 Pa to 400 Pa, in particular from 90 Pa to 300 Pa, in particular from 100 Pa to 200 Pa. In this context, the storage modulus is measured according to the frequency sweep strain controlled method with a Kinexus rheometer (NETZSCH GmbH, Selb, Germany). A parallel plate, 8 mm in diameter was used for all the measurements, with the average normal force maintained at 0.1 N at 25 _{°}C and 37 _{°}C. The data was analyzed by an oscillatory frequency sweep strain-controlled test with 1% strain (which is obtained from a linear viscoelastic range of an amplitude sweep test) and the reported storage modulus (G') of a rigid hydrogel was picked at 0.3Hz.

Briefly, a shear force is applied to the hydrogel to conduct the experiment. This results in a shear modulus value. There are two types of modulus behaviors observed in the shear force mode. The first is called storage modulus, which indicates how elastic the hydrogel sample is or how much energy is stored in the hydrogel, assuming that the hydrogel behaves like a solid. The other type is called loss modulus, which indicates how viscous the hydrogel sample is, assuming that the hydrogel behaves like a liquid. Presently, the inventors focused on measuring the storage modulus as it is the essential data of hydrogels due to a crosslink network being responsible for energy storage. Since the storage modulus is determined by a shear force experiment, the storage modulus is sometimes also referred to as shear modulus.

In an aspect, the present invention relates to the use of a hydrogel according to any of the preceding explanations for binding viruses in vitro.

In an embodiment, the viruses to be bound by the hydrogel are influenza viruses, immunodeficiency virus type-1 (HIV), dengue viruses, or coronaviruses, such as severe acute respiratory syndrome coronavirus 2 (SARS-CoV-2).

In an embodiment, the viruses to be bound by the hydrogel are herpes simplex viruses (HSV-1).

In an aspect, the present invention relates to the use of a hydrogel according to the preceding explanations as matrix for cultivating and/or expanding cells and/or organoids in vitro. Particularly appropriate cells for such cell culture/expansion technique are stem cells.

In an aspect, the present invention relates to the medical use of a hydrogel according to the preceding explanations in antiviral therapy of humans or animals, i.e. in in-vivo antiviral therapy. Due to its viral binding capacity, the hydrogel can well be used in-vivo as antiviral component.

In an aspect, the present invention relates to a medical method for binding viruses in vivo, i.e. to an antiviral therapy. This method employs the hydrogel according to the preceding explanations as virus binding agent. The hydrogel is administered to a person in need thereof to be subsequently able to bind viruses prior to or upon entering the patient's body. Due to the antiviral activity of the hydrogel, severe infections with different types of viruses can be avoided by this method.

In an aspect, the present invention relates to the medical use of a hydrogel according to the preceding explanations as mucus replacement, as cartilage replacement, or as synovial fluid replacement in a patient in need thereof. The rheological and viscoelastic properties of the hydrogel can be finely tuned to match the corresponding properties of mucus, cartilage, or synovial fluid. This opens a wide scope of applications of the presently claimed and described hydrogel. In addition, the respective mucus replacement, cartilage replacement, or synovial fluid replacement exhibits antiviral properties and can thus prevent infections and assist healing processes.

In an aspect, the present invention relates to a pharmaceutical composition comprising a hydrogel according to the above explanations. Such a pharmaceutical composition can be provided, e.g., in form of a lip gel or lipstick. In such a case, the pharmaceutical composition is particularly appropriate to be used for treating or preventing an infection with herpes viruses, such as herpes simplex viruses (HSV-1). Other appropriate dosage forms of such a pharmaceutical composition are creams, ointments and gels (irrespective of their intended site of use on the human or animal body).

In an aspect, the present invention relates to a method for manufacturing a hydrogel according to the preceding explanations. This method comprises the step of mixing an aqueous solution of a dendritic polyglycerol maleimide corresponding to general formula (IV) with an aqueous solution of a poly(ethylene glycol) dithiol corresponding to general formula (V):

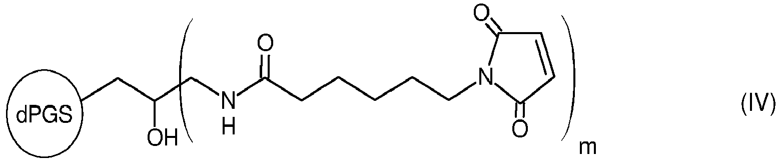

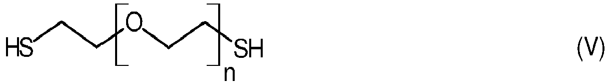

In this context, the abbreviations have the following meanings:
dPGS is a dendritic polyglycerol core comprising terminal hydroxyl groups and terminal sulfate or sulfonate substituents, wherein a degree of substitution of the dendritic polyglycerol core with sulfate or sulfonate substituents lies in a range from 10 % to 98 %,
m is 2 % to 10 %, in particular from 3 % to 9 %, in particular from 4 % to 8 %, in particular from 5 % to 7 %, of all terminal groups of the dendritic polyglycerol core that are not substituted with the sulfate or sulfonate substituents,
n is a number lying in range from 1 to 1000, in particular from 2 to 900, in particular from 3 to 800, in particular from 4 to 700, in particular from 5 to 600, in particular from 6 to 500, in particular from 7 to 400, in particular from 8 to 300, in particular from 9 to 200, in particular from 10 to 100, in particular from 20 to 90, in particular from 30 to 80, in particular from 40 to 60.

In an embodiment, a molar ratio between the poly(ethylene glycol) dithiol and the dendritic polyglycerol maleimide lies in a range from 1:1.5 to 9:1, in particular from 2:1 to 9:1, in particular from 1.5:1 to 7:1, in particular from 1.2:1 to 5:1, in particular from 1:1 to 3:1, in particular from 1:1.5 to 2:1.

In an embodiment, the dendritic polyglycerol maleimide is manufactured by reacting a dendritic polyglycerol amine having a dendritic polyglycerol core comprising terminal hydroxyl groups and terminal sulfate or sulfonate substituents with 6-maleimidohexanoic acid, N-hydroxysuccinimide and N,N'-diisopropylcarbodiimide. In this context, a degree of substitution of the dendritic polyglycerol core with sulfate or sulfonate substituents lies in a range from 10 % to 98 %, in particular from 15 % to 95 %, in particular from 20 % to 90 %, in particular from 30 % to 85 %, in particular from 40 % to 70 %, in particular from 50 % to 60 %, in particular from 70 % to 98 %, in particular from 75 % to 95 %, in particular from 80 % to 90 %.

In an embodiment, the dendritic polyglycerol amine carries sulfate substituents. It can thus be denoted as dendritic polyglycerol sulfate (dPGS) amine. The dPGS amine is manufactured by mesylating a non-substituted dendritic polyglycerol (dPG) to obtain a mesylated dendritic polyglycerol, adding an azide such as sodium azide to the mesylated dendritic polyglycerol to form a dendritic polyglycerol azide, and reducing and sulfating the dendritic polyglycerol azide with triphenylphosphine and sulfur trioxide to obtain the dPGS, i.e., the dendritic polyglycerol amine carrying sulfate substituents.

All embodiments of the hydrogel can be combined in any desired way and can be transferred either individually or in any arbitrary combination to the described uses, to the described pharmaceutical composition, and the described methods. Likewise, all embodiments of the described uses can be combined in any desired way and can be transferred either individually or in any arbitrary combination to the hydrogel, to the pharmaceutical composition, and to the methods. All embodiments of the pharmaceutical composition can be combined in any desired way and can be transferred either individually or in any arbitrary combination to the hydrogel, to its uses, and to the methods. Finally, all embodiments of the different methods can be combined in any desired way and can be transferred either individually or in any arbitrary combination to the hydrogel, to the pharmaceutical composition, to the uses or to the respective other method.

Further details of aspects of the present invention will be explained in the following making reference to exemplary embodiments and accompanying Figures. In the Figures:
- Figure 1A: shows a reaction scheme of the synthesis of poly(ethylene glycol) dithiol;
- Figure 1B: shows a reaction scheme of the synthesis of dendritic polyglycerol (dPG) maleimide;
- Figure 1C: shows a reaction scheme of the synthesis of dendritic polyglycerol sulfate (dPGS) maleimide;
- Figure 2A: shows a pictorial representation of an embodiment of a hydrogel and its internal matrix structure;
- Figure 2B: illustrates the rheological properties of a hydrogel made from polyglycerol sulfate maleimide and poly(ethylene glycol) dithiol (HGS) having a gel concentration of 8 % (HGS 8 %);
- Figure 2C: illustrates the rheological properties of HGS 5 %;
- Figure 2D: illustrates the rheological properties of HGS 4 %;
- Figure 2E: illustrates the rheological properties of HGS 3 %;
- Figure 3A: shows the storage (G') and loss (G") moduli as a function of radial frequency (ω) of hydrogels made from polyglycerol maleimide and poly(ethylene glycol) dithiol (HG) at 37 ºC;
- Figure 3B: shows the storage (G') and loss (G") moduli as a function of radial frequency (ω) of hydrogels made from polyglycerol sulfate maleimide and poly(ethylene glycol) dithiol (HGS) at 37 ºC;
- Figure 4A: shows the storage (G') and loss (G") moduli as a function of radial frequency (ω) of swollen hydrogels made from polyglycerol maleimide and poly(ethylene glycol) dithiol (HG) at 37 ºC;
- Figure 4B: shows the storage (G') and loss (G") moduli as a function of radial frequency (ω) of swollen hydrogels made from polyglycerol sulfate maleimide and poly(ethylene glycol) dithiol (HGS) at 37 ºC;
- Figure 5A: shows the results of cytotoxicity tests conducted for dPG maleimide and dPGS maleimide with cells of the Vero-E6 cell line;
- Figure 5B: shows the results of cytotoxicity tests conducted for dPG maleimide and dPGS maleimide with cells of the A549 cell line;
- Figure 5C: shows the results of cytotoxicity tests conducted for dPG maleimide and dPGS maleimide with cells of the HBE cell line; and
- Figure 6: shows the efficacy of HSV-1 binding by different hydrogels.

### Synthesis of gel components

### Synthesis of PEG dithiol

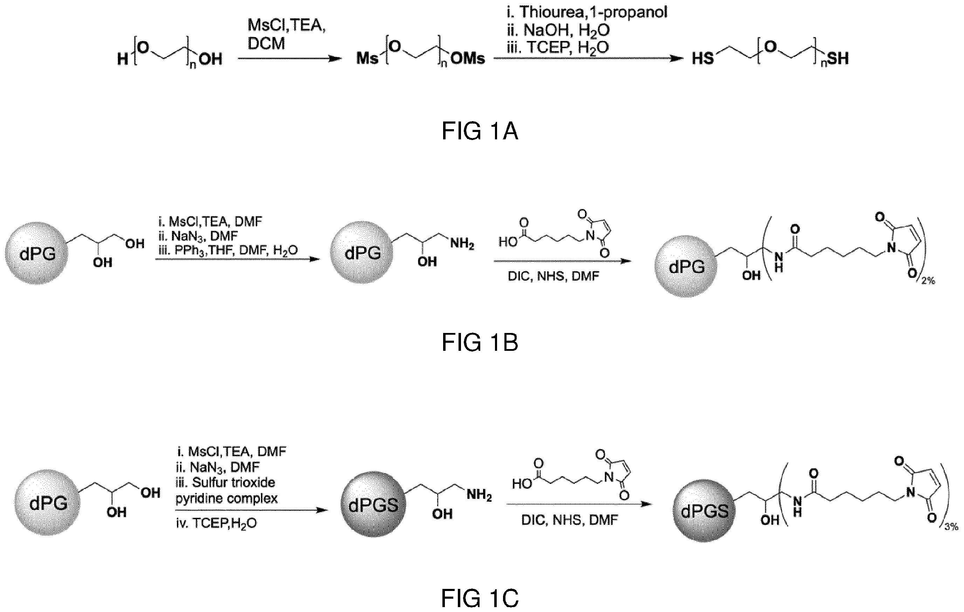

Commercially available PEG with a molecular weight of 6 kDa was first mesylated and subsequently purified by precipitation resulting in PEG(OMs)₂ in the form of a white powder. In order to synthesize PEG dithiol, PEG(OMs)₂ was first allowed to react with dithiourea, wherein the intermediate diisothiouronium PEG was formed. This was immediately followed by basic hydrolysis to finally obtain PEG dithiol, PEG(SH)₂. Tris(2-carboxyethyl)phosphine (TCEP) was added as reducing agent at this point, and purification thereafter by extraction and precipitation resulted in pure PEG(SH)2, as confirmed by ¹H NMR spectroscopy. The synthesis is depicted in **Figure 1A****.**

### Synthesis of dPG maleimide

Dendritic polyglycerol (dPG) was synthesized following the procedure in literature. dPG maleimide was synthesized in four steps. First of all, dPG was mesylated. To the mesylated product, sodium azide was added to form dPGN₃ following a substitution reaction. The resulting mixture containing the product was purified by dialysis. dPG amine was synthesized by the reduction of dPGN₃ with triphenylphosphine (TPP). DCM wash and dialysis of the crude product resulted in pure dPGNH2. This was corroborated by ¹H NMR and ¹³C NMR spectroscopy results. Finally, the amine group was reacted with the active N-hydroxysuccinimide (NHS) ester formed by the reaction of NHS, diisopropylcarbodiimide (DIC) and maleimidohexanoic acid resulting in the formation of dPG maleimide. Pure dPG maleimide was obtained by dialysis of the crude product against water, confirmed thereafter by ¹H NMR studies. The synthetic steps are shown in **Figure 1B****.**

### Synthesis of dPGS maleimide

Dendritic polyglycerol sulfate (dPGS) maleimide was prepared in a similar fashion to dPG maleimide, as depicted in **Figure 1C****.** dPGN₃ was synthesized in the same manner as mentioned above. To the same reaction mixture, sulfur trioxide pyridine complex was added and subsequently the crude mixture was first neutralized and then purified by dialyzing it against first brine, followed by water. The obtained product was then reduced and purified by dialysis. The consequent dried dPGS amine was obtained in the form of a yellow powder, the purity of which was determined by ¹H NMR spectroscopy. Afterwards, dPGS maleimide was synthesized by the reaction of dPGS amine with NHS, DIC and maleimidohexanoic acid. After purification by dialysis against water, the final product was obtained as a pale yellow powder. The synthetic purity and 84% degree of sulfation were confirmed by ¹H NMR spectroscopy and elemental analysis, respectively.

### 2.2 Synthesis of gels

Two types of gel series were constructed. dPG maleimide gels, i.e. gels represented by "HG" were created from PEG dithiol and dPG maleimide. Further, to identify the importance of the decorating functional groups, dPGS maleimide gels (represented by "HGS") were synthesized from the reaction between PEG dithiol and dPGS maleimide. The synthetic procedure was rapid and simple: the PBS solutions of two gel components as well as an additional amount of PBS were vortexed together so as to amount to a total of 100 µL gel volume in the ratios indicated in **Table 1.** Further, the gels at 37 ºC were allowed to swell for one hour, and oscillatory rheology experiments were carried out to determine their mechanical properties and pore sizes. The ratio of the two reacting components in this case, PEG-dithiol and dPG maleimide was maintained to be the same as 2.5:1 respectively, in every gel decreasing the overall gel components concentration.

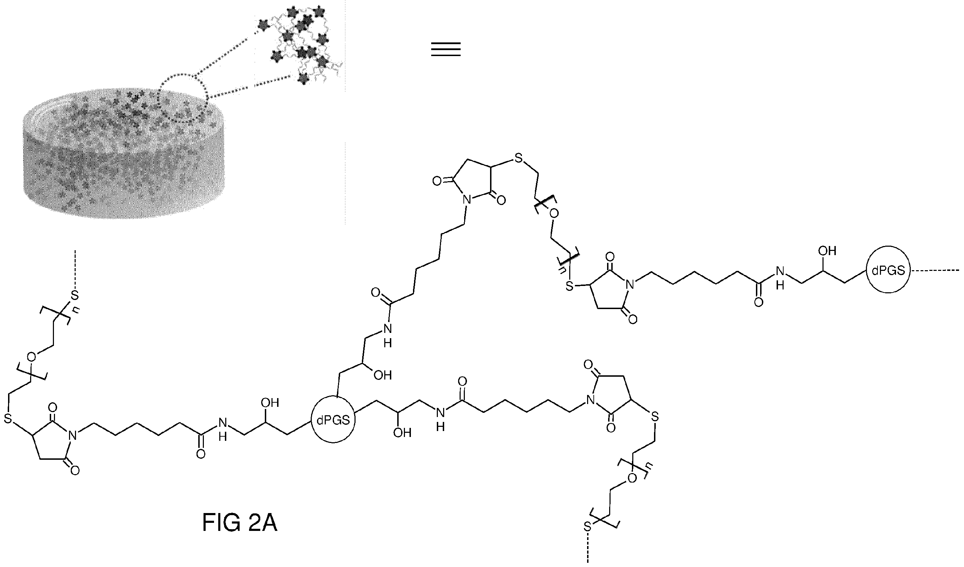

**Figure 2A** shows a pictorial representation of the dPGS maleimide hydrogel and its internal matrix structure (enlarged structure). The dPG units (dark grey) are decorated with maleimide groups (black) which then react with PEG dithiol (light grey) in a Michael-click reaction to result in the formation of the hydrogel.

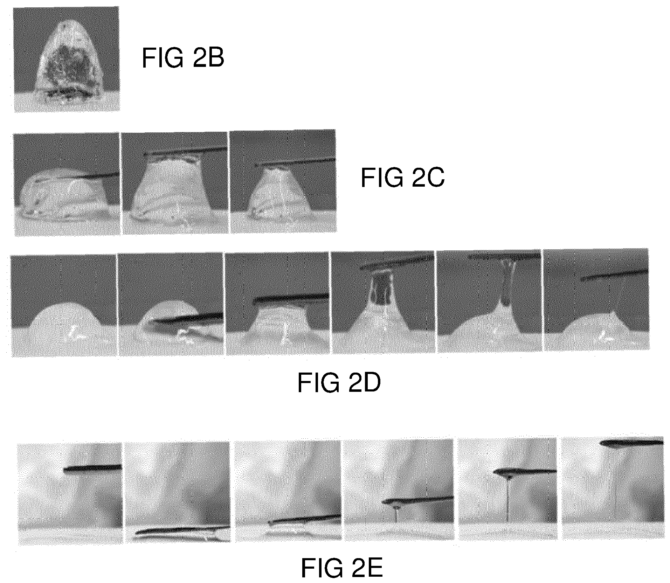

Increasing dilution of gel components allowed a decrease in the number of cross-links, and thus gel elasticity decreased and conversely its viscosity increased, as can also be clearly visualized in the **Figure 2B** - **2E****,** showing sulfated hydrogels with a gel concentration of 8 %

**(****Figure 2B****),** 5 % **(****Figure 2C****),** 4 % (**Figure 2D****),** and 3 % (**Figure 2E****).** Each gel displays a clear difference in its physical properties, which are dependent upon its rheological properties. Gel HGS 8% **(****Figure 2B****)** maintains a firm structure, while the viscous trait dominates in gel HGS 3% **(****Figure 2E****).**

Initially, with an overall gel concentration of 8%, the rigidity of the HGS 8% gels **(****Figure 2B****)** is very high. For this reason, it maintains a quite rigid structure which does not flow within the measured time period.[14] Spinnability or the ability of thread formation is a unique property arising from non-Newtonian flow, where the existence of both elastic and viscous properties is an important prerequisite. As such, spinnability increases in viscoelastic compounds with an increase in viscosity.[14] As the gel concentration decreases from 5% to 3% **(****Figure 2C - 2E****),** their viscosity increases. Gel HGS 5% **(****Figure 2C****)** is also quite elastic as it hardly flows when pressure is applied and quickly recoils to its previous position as pressure is removed. It does not display spinnability suggesting a very low viscous characteristic as well. Conversely gels HGS 4% and HGS 3% **(****Figures 2D** and **2E****,** respectively), are both soft flowable gels. They are not able to maintain their conformation with the increasing pressure. In fact, HGS 3% appears to be a viscous liquid; however, as evidenced by its very high spinnability, it maintains some elasticity and thus its network structure as well.

**Table 1. Composition of different non-sulfated (HG) and sulfated (HGS) gel types depicting the amounts and ratios of the gel components (10% w/v PEG, 16% w/v dPG and 10% w/v dPGS) used per 100 µL volume.**

| **Hydrogel** | **Gel type** | **PEG: dPG/dPGS mole ratio** | **Gel components (volume, µL)** | | **PBS (µL)** | **Total gel volume (µL)** | **Gel concentration (% w/v)** |
|---|---|---|---|---|---|---|---|
| | | | **PEG** | **dPG/ dPGS** | | | |
| HG 8 % | dPG maleimide | 2.5:1 | 48 | 20 | 32 | 100 | 8.0 |
| HG 7 % | dPG maleimide | 2.5:1 | 42 | 17.5 | 40.5 | 100 | 7.0 |
| HG 6 % | dPG maleimide | 2.5:1 | 36 | 15 | 49 | 100 | 6.0 |
| HG 5 % | dPG maleimide | 2.5:1 | 30 | 12.5 | 57.5 | 100 | 5.0 |
| HG4% | dPG maleimide | 2.5:1 | 24 | 10 | 66 | 100 | 4.0 |
| HGS 8 % | dPGS maleimide | 2.5:1 | 34.3 | 45.7 | 20 | 100 | 8.0 |
| HGS 6 % | dPGS maleimide | 2.5:1 | 25.8 | 34.2 | 40 | 100 | 6.0 |
| HGS 5 % | dPGS maleimide | 2.5:1 | 21.5 | 28.5 | 50 | 100 | 5.0 |
| HGS 4 % | dPGS maleimide | 2.5:1 | 17.2 | 22.8 | 60 | 100 | 4.0 |
| HGS 3 % | dPGS maleimide | 2.5:1 | 12.9 | 17.1 | 70 | 100 | 3.0 |

### Rheology

### Oscillatory Rheology

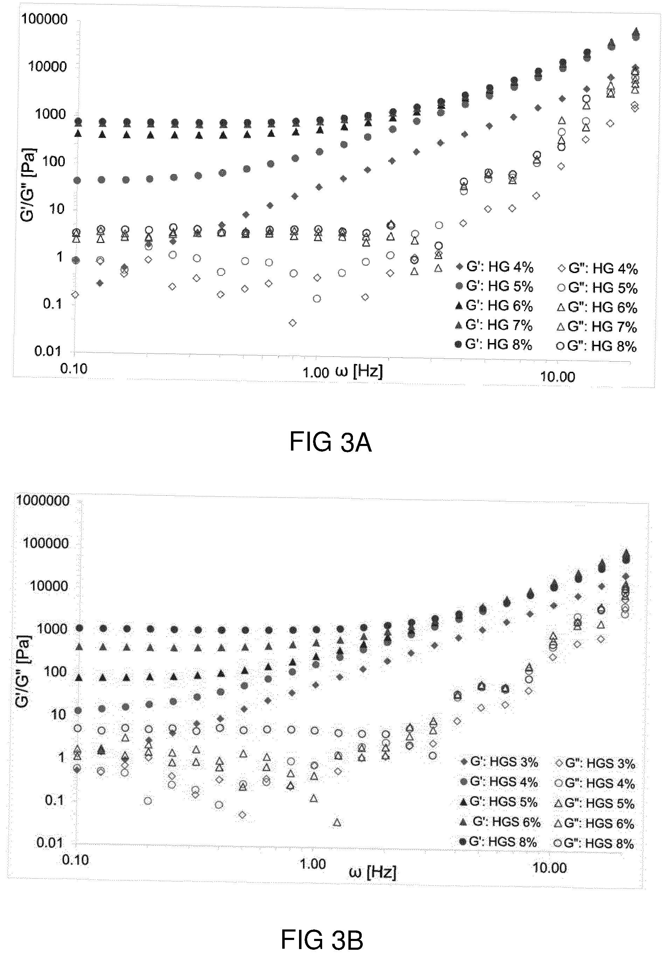

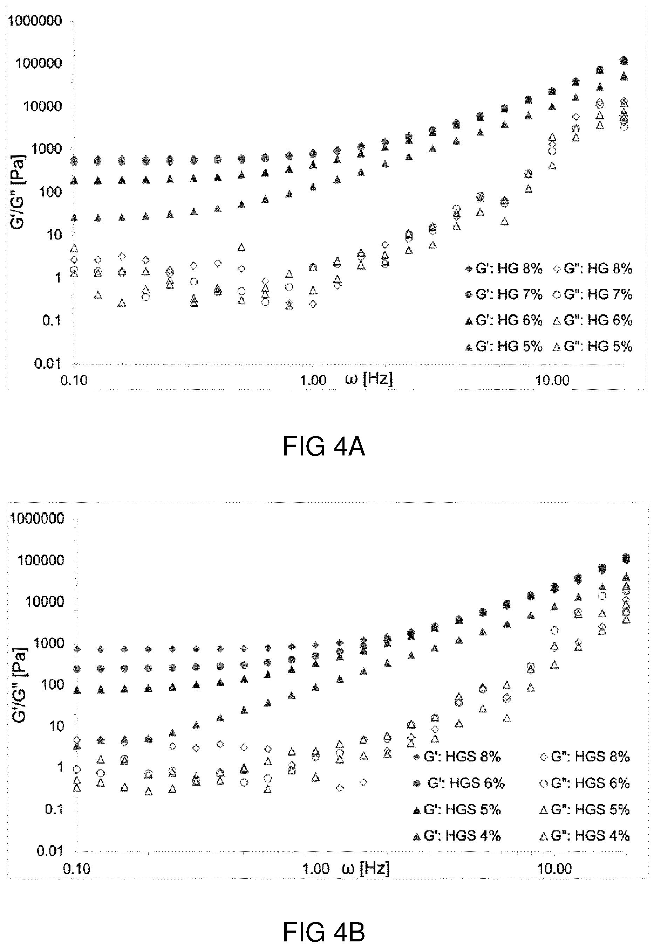

A substance's viscoelastic properties are essentially indicative of its mechanical properties and can be determined by oscillatory rheology experiments. A strain-sweep test was carried out over the entire series to establish the linear viscoelastic region (LVE) so that consecutive oscillatory rheology experiments would be conducted in this region. These viscoelastic experiments allowed the deduction of the storage modulus, G' and the loss modulus, G" as a function of the radial frequency, *ω*. Moreover, the experiments were conducted at 25 ºC and at the physiological temperature 37 ºC, where the viscoelastic properties of the swollen gels were measured as well. The results of the experiments at 37 ºC are shown in **Figures 3A** and **3B****,** while the results of the swollen gels are shown in **Figures 4A** and **4B****.**

The rheological behavior of the non-sulfated gels "HG" is shown in **Figures 3A** and **4A** at 37 ºC in the initial and swollen states after one-hour incubation with excess water, respectively. All the gels displayed notable elastic-dominated behavior, thus providing ample proof of the stability of the crosslinks. When comparing the behavior of these gels with their behavior at 25 ºC, the difference caused by the increase in temperature to the physiological temperature is negligible. Interestingly, the swollen state causes a notable difference in the viscoelastic behavior in all these gels. As can be seen in **Table 2,** there is a general trend of decrease in the elastic character of the gels as the gels become more swollen.

**Table 2. Shear modulus G₀ and estimated mesh size of hydrogel series HG and HGS in the initial and swollen states at 37 ºC, wherein the initial state refers to the hydrogel as soon as it forms after mixing, and the swollen state refers to the hydrogel after one hour of incubation with excess water.**

| | **37 °C (initial state)** | | **37 °C (swollen state)** | |
|---|---|---|---|---|
| **Hydrogel** | **Shear modulus [G₀/Pa]** | **Mesh size [*ξ*/nm]** | **Shear modulus [G₀/Pa]** | **Mesh size [*ξ*/nm]** |
| HG4% | 5 | 91 | - | - |
| HG 5 % | 67 | 39 | 43 | 46 |
| HG 6 % | 433 | 21 | 234 | 26 |
| HG 7 % | 716 | 18 | 556 | 19 |
| HG 8 % | 766 | 18 | 630 | 19 |
| HGS 3 % | 10 | 76 | - | - |
| HGS 4 % | 40 | 47 | 17 | 62 |
| HGS 5 % | 111 | 33 | 118 | 33 |
| HGS 6 % | 442 | 21 | 283 | 24 |
| HGS 8 % | 1119 | 15 | 748 | 18 |

The viscoelastic behavior of the sulfated "HGS" gels at 37 ºC is shown in **Figures 3B** and **4B****.** All gels in this series can be seen to behave in a similar fashion. An increase in both moduli is seen for all cases with increasing frequency, as expected. Further, as with the HG series, all HGS gels are elastic-dominated, indicating the existence of permanent crosslinking in a solid gel matrix. In comparison to the non-sulfated HGs, the general trend of elasticity was more towards the lower end. This can be explained by the electrostatic repulsion present within these gels contributed by the sulfate groups. An increase in temperature from 25 ºC to 37 ºC did not affect the rheological properties in this case. However, a significant decrease was observed in the elastic modulus of all the sulfated gels when they were in the swollen state, as can be seen in **Table 2.** This occurs due to the increase in the general pore size with swelling and this effect is particularly pronounced due to the high-water attraction of charged sulfate groups, exemplified by gel HGS 4% wherein a more than two times decrease of the elastic modulus was observed in the swollen state comparison to the initial state at 37 ºC.

Moreover, the change in the viscoelastic properties are more distinct in this case in comparison to the non-sulfated gels. Out of all the sulfated hydrogels, the HGS 3% was the most viscous, with its shear modulus in the non-swollen state being only 10 Pa. The rheological behavior of the non-swollen HGS 3% network was consistent with the rest of the hydrogels in the series. For most part the elastic modulus remained higher than the viscous modulus, except for in the lower frequency range in the very beginning, as shown in **Figure 3B****.**

The existence of a stable network was also seen by spinnability. As with all other gels in this series, it is expected that its shear modulus would decrease further with swelling. For this reason, its behavior in the swollen state could not be determined, as the viscosity of the gel was quite high and therefore became miscible on the addition of PBS. However, the nature of the crosslinks remained stable as the swollen gels showed spinnability as well. In fact, the HGS 3% also show some similarities to naturally occurring gel mucus. As their shear modulus in the swollen state would be lower than 10 Pa, they would be in the approximate shear modulus range of healthy lung mucus, which is about 1 - 2 Pa.[15] Moreover, lung mucus also exhibits a similar rheological pattern, maintaining a plateau at lower frequencies, and then an increase above 10 Hz. The plateau modulus is the frequency range wherein an overall linear behavior of the storage and loss moduli is seen. Materials like hydrogels possess a complex rheological profile owing to the presence of a defined internal structure. While the macrorheology, or its bulk rheological properties such as viscoelasticity govern its functions such as lubrication and interaction with surfaces, its microrheological properties determine the diffusion behavior of small components like pathogens or drugs, within the hydrogel matrix. While a larger mesh size allows free diffusion of a smaller component, when the two are comparable, steric hindrance on movement becomes significant. Thus precise control over the mesh size is pertinent to its application.[16]

In order to calculate the mesh size, the G' value at 0.3 Hz frequency was chosen and then substituted in the simplified equation G' = *kT*/*ξ*³*,* where k is the Boltzmann constant, T is the temperature and *ξ* is the mesh size.[17]-[19] As a rule, the mesh sizes increased as the crosslinking density decreased.[20] The lowest mesh sizes would therefore be predicted as corresponding to the least elastic hydrogel. Indeed, among the initially formed sulfated and non-sulfated hydrogel HG 4% and HGS 3% had the largest mesh size at approximately 91 and 76 nm, respectively owing to the low crosslinking density. It was found that HSV measuring 180 nm in size were slowed down in mucus with mesh size of -100 nm, with up to a 1000 fold decrease in compared to water.[21] The mesh size of the sulfated HGS 3% and 4% gels approaches this and can therefore can be considered as suitable candidates for hindering HSV.

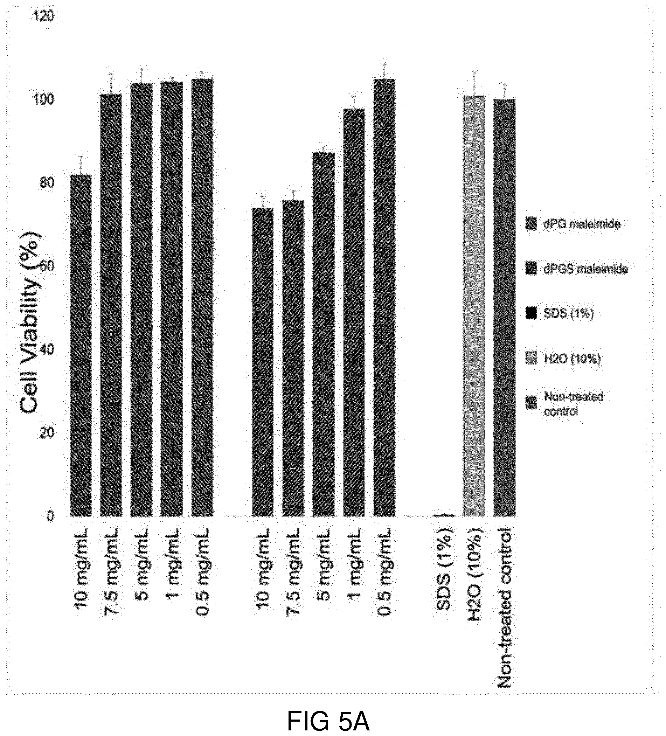

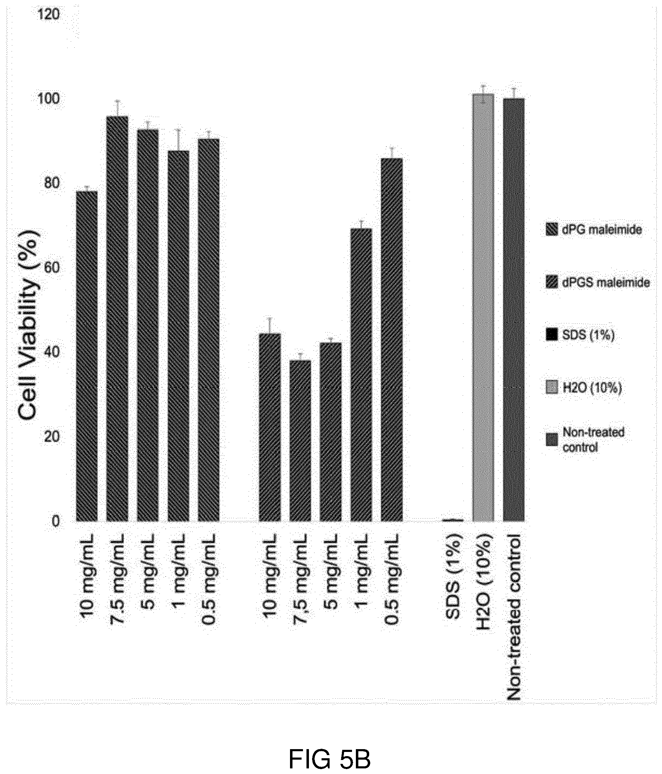

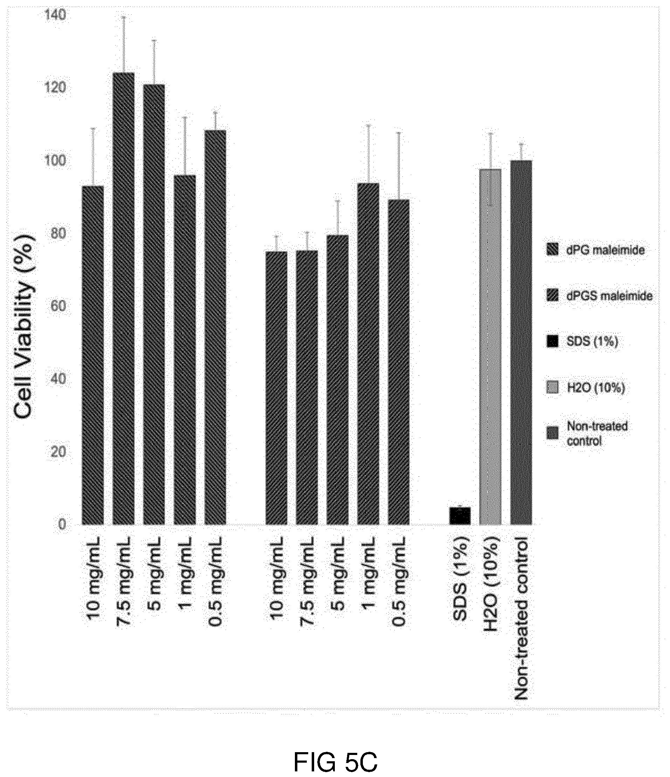

The cytotoxicity tests of gel components were performed against different cell types with CCK-8 kit. Vero E6 cell line (Figure **5A**) was used, which is generally applied for infection assays and virus propagation studies. Moreover, the human lung cell lines A549 and HBE were also employed to diversify the study (Figures **5B** and **5C****,** respectively). In all diagrams of Figures 5A, 5B and 5C, the first five bars represent the results of dPG maleimide, whereas the second five bars represent the results of dPGS maleimide. A slight decrease in the cell viability was observed with the addition of sulfated compounds, as expected. A high tolerance of the gel components for all the cell lines was observed, and the tolerance of dPGS decreased only at the concentration > 1 mg/mL. dPG maleimide showed high cell viability throughout the tested range for all cell lines. A549, Vero E6, as well as the HeLa cell lines were treated with PEG dithiol and a high tolerance was noted in every case. Overall, all the experiments proved a high tolerance for all the gel components involved rendering them safe to be applied for future treatments.

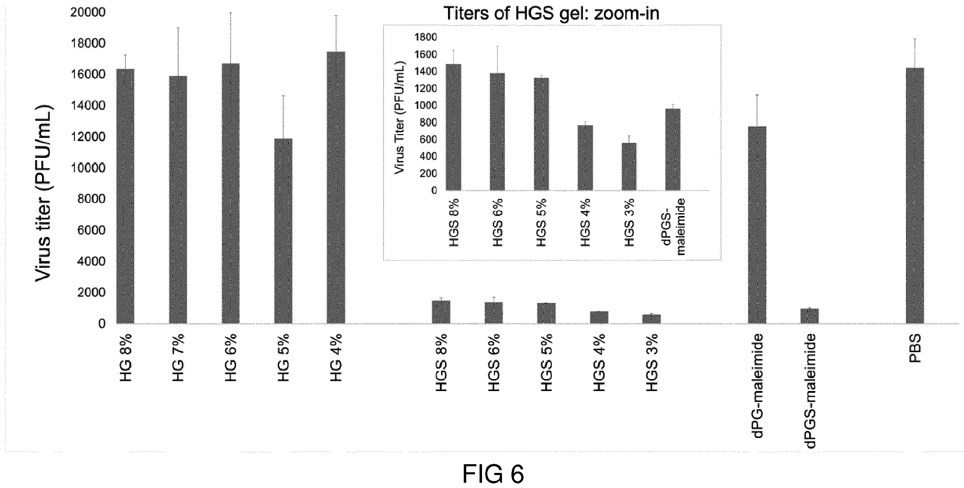

The HSV-1 binding of the hydrogels was established by plaque reduction assays. For this purpose, the hydrogel was initially incubated with the virus solution with moderate shaking for 1h. Afterward, the number of viral particles in the supernatant is titrated by plaque assays on Vero E6 cells. The binding with virus was revealed by reduced virus titer in the supernatant as shown in **Figure 6** that characterizes the virus titers after treatment with various samples. The insert in **Figure 6** shows the virus titers of gel series HGS, as well as dPGS-maleimide. All values are expressed as mean ± SD, n=3. dPGS-maleimide (5% w/v) and dPG-maleimide (8% w/v) were applied in the HSV binding assay.

No significant reduction in the HSV titer with the non-sulfated control gels or gel component dPG-maleimide was observed. The sulfated gel types (HGS 8%, HGS 6%, HGS 5%, HGS 4%, HGS 3%) showed much higher binding abilities of up to 30 times higher than their non-sulfated counterparts. HSV viruses electrostatically bind with heparan sulfate on the host cells through their surface glycoprotein. Due to the presence of sulfate groups in the HGS gels, virus binding became significantly more efficacious in comparison to HG gels. This was observed not only for every hydrogel but also for the individual sulfated and non-sulfated gel components (dPG-maleimide and dPGS-maleimide). However, as the gels HGS 8% - 5% are quite stiff, their sulfate groups are less exposed even after swelling, and thus do not interact with as many HSV particles as the softer gels would. This has a negative effect on their binding performance, as can be seen in **Figure 6****.**

The effects of network structure on virus binding were revealed by the comparison among sulfated hydrogels with different stiffness. The more viscous gel HGS 3% being slightly better than the HGS 4%, showed a higher HSV interaction than the more elastic gels HGS 5% - 8% in the series. The remaining virus titer in the supernatant treated with HGS 3% gel was 560 PFU/mL whereas for HGS 8%, the remaining virus titer was still 1,483 PFU/mL. The loosely bound network in the more flexible sulfated hydrogels might allow the exposition of more sulfate groups while binding with the virus and thus is definitely a plus for HSV binding and inhibition.

Sulfated hydrogels HGS were compared with the 2.3 mM sulfated gel component dPGS-maleimide (-4.6% w/v). The concentration of sulfated gel component in the HGS 4% (-1.1 mM dPGS) and HGS 3% (-0.9 mM dPGS) are 2-2.5 times lower and all sulfate groups are not fully exposed because of 3D network formation, still the performance of these gel types were slightly higher than the dPGS-maleimide itself because of large contact surface area with the virus and high flexibility. Thus, sulfated gels proved to be the most compelling candidates for HSV-1 binding. Moreover, the network structure also played a significant role in this study, where higher flexibility allowed higher binding capacity.

### Conclusion

The present invention demonstrates the flexibility of a sulfated hydrogel network as an important parameter for its ability to bind HSV-1 virus. In order to deduce the best candidate, polyether-based hydrogels were prepared using thiol-maleimide click chemistry based on dPG-maleimide as a crosslinker and PEG-dithiol as the linear component. Two sets of hydrogels were compared, distinguished by the presence of hydroxyl groups in one series and sulfate groups in the other series. Furthermore, hydrogels were prepared within each series such that their flexibility was tuned reduction of the linear component. A range of flexibilities with shear moduli between 1 and 1200 Pa were achieved. The presence of sulfate groups on hydrogels is crucial for HSV binding, rheology dependent parameters also played an important role. The sulfated hydrogels show 10-30 times stronger HSV binding than the non-sulfated controls. Furthermore, gels followed a general trend of having higher virus titer reduction as their flexibilities increased. Notably, HGS 3% proved to be the most suitable virus-binding candidate, showing an even higher binding capacity than the highly sulfated dendritic crosslinker dPGS-maleimide. These polysulfated hydrogel networks can mimic the antiviral function of mucus.

### Experimental details

### Materials

All chemicals were purchased from Merck KGaA, Darmstadt, Germany and/or its affiliates and used without any further purification, unless otherwise stated. The solvents used herein, i.e., diethyl ether (100%) and *N,N*-Dimethylformamide (99.8%) were bought from VWR chemicals and Acros Organics, respectively while DCM (99%) and ethyl acetate were both obtained from Fischer Scientific. Sodium hydroxide in the form of pellets, as well as in a 99.5% solution, were also procured from Fischer Scientific. Triphenylphosphine (99%) was purchased from Alfa Aesar. dPG of approximately 6 kDa average molecular weight was prepared as previously reported [22-24] with the improved method.[25] For purification carried out with dialysis, Spectra Por dialysis tubing (MWCO = 2000 g/mol) (Carl Roth GmbH, Karlsruhe, Germany). Cell viability assays were performed with a CCK-8 Kit from Sigma Aldrich according to the manufacturing instructions. A549, HBE, HeLa, and Vero E6 cells were obtained from Leibniz-Institut DSMZ - Deutsche Sammlung von Mikroorganismen und Zellkulturen GmbH and cultured in DMEM supplemented with 10% (v/v) FBS, 100 U/mL penicillin and 100 µg/mL streptomycin.

### Instrumentals

The Jeol Eclipse 500 MHz (Tokyo, Japan) or a Bruker AVANCE III 700 MHz spectrometer (Billerica, MA, USA) instruments were used to measure all the NMR spectra of all the compounds (1H and 13C) reported here were recorded at 300 K. Chemical shifts δ were reported in ppm and the deuterated solvent peak was used as a standard. Vario EL CHNS element analyzer (Elementar Analysensysteme GmbH (Langenselbold, Germany)) was used to carry out the elemental analysis of all relevant compounds reported in this work. All the rheology data reported here was measured and characterized by the Kinexus rheometer (NETZSCH GmbH, Selb, Germany). A parallel plate, 8 mm in diameter was used for all the measurements, with the average normal force maintained at 0.1 N at 25 _{°}C and 37 _{°}C. The data were analyzed by an oscillatory frequency sweep strain-controlled test with 1% strain (which is obtained from a linear viscoelastic range of an amplitude sweep test) and the reported storage modulus (G') of a rigid hydrogel were picked at 0.3Hz.

### Synthesis of PEG(OMs)₂

Dried PEG (20 g, 3.3 mmol ,1 eq., 6 kDa) was dissolved in a dichloromethane (DCM) solution (100 mL), and subsequently cooled down in an ice bath. Then triethylamine (TEA, 2.77 mL, 20 mmol, 6 eq.) was added to the solution, followed by the dropwise addition of methanesulfonyl chloride (1.03 mL, 13.3 mmol, 4 eq.). The reaction was allowed to run overnight. Afterwards, the crude product was purified; the DCM layer was washed thrice with brine before drying it with Na2SO4. It was then precipitated in cooled diethyl ether. The purified precipitate was then allowed to dry overnight under vacuum, finally resulting in a white powder with 95% isolated yield. ¹H NMR: (500 MHz, CDCl3, δ (ppm)): 3.07 (3H, s), 3.48 - 3.78 (m), 4.37 (2H, t)

### Synthesis of PEG dithiol (PEG(SH)₂)

PEG(OMs)₂ (19 g, 3.2 mmol, 1 eq.) and thiourea (1.02 g, 13.3 mmol, 4 eq.) were added to a solution of 1-propanol. The solution was refluxed overnight to obtain diisothiouronium PEG as the intermediate product. Without any further purification, 1-propanol was immediately removed from the intermediate, followed by the addition of NaOH (0.53 g, 13.3 mmol, 4 eq.) and water (100 mL). The reaction mixture was allowed to reflux overnight. Afterwards, tris(2-carboxyethyl)phosphine (TCEP, 1.67 g, 6.7 mmol, 2 eq.) was added to the mixture and stirred for 2 h prior to the purification. To purify, NaCl was added to the mixture until the point of saturation, followed by the precipitation of the product was extracted three times into DCM. The DCM layer was then dried by Na₂SO₄, after which it was precipitated in cooled diethyl ether. The precipitate was dried *in vacuo* overnight. PEG dithiol was obtained as a pale yellowish powder in 88% isolated yield. ¹H NMR (500 MHz, CDCl3, δ (ppm)): 1.59 (1H, t), 2.69 (2H, quat), 3.48 - 3.78 (m). Elemental analysis; N = 0.13; C = 54.24; S = 2.02; H = 8.47

### Synthesis of dPGNH₂

Dried dPG (5 g, 0.5 mmol, 1 eq.) and TEA (0.7 mL, 5 mmol, 10 eq.) were added to a solution of *N,N*-dimethyl formamide (DMF, 50 mL) and the reaction mixture was subsequently cooled using an ice bath. Methanesulfonyl chloride (0.31 mL, 4 mmol, 8 eq. to target roughly 5% mesyl groups on dPG) was added dropwise to the stirring mixture. The reaction mixture was then stirred overnight. NaN₃ (0.65 g, 10 mmol, 20 eq.) was then added to the reaction flask and thereafter heated at 60 _{°}C for 2 days. Afterwards, the crude mixture was purified in water by dialysis (MWCO = 2 kDa) for 2 days. After purification, water was first removed from the flask and DMF (40 mL) was then added to it. Separately a tetrahydrofuran (THF, 30 mL) solution of triphenylphosphine (TPP, 3.28 g, 12.5 mmol, 25 eq.) was prepared in another flask. The contents of the latter were then added gradually to the former DMF solution flask. The reaction flask was allowed to stir overnight, with additional care that phase separation did not take place. Afterwards, water (5 mL) was added to the reaction mixture and stirred again overnight at room temperature overnight. Finally, the product was purified by a DCM wash, repeated three times and later dialyzed against water (MWCO = 2 kDa) for 2 d. The product obtained was then dried and collected as a pale yellowish liquid with a honey-like consistency in 70% isolated yield. ¹H NMR (700 MHz, D2O, δ (ppm)): 0.90 (3H, broad s, initiator backbone), 1.39 (2H, broad s, initiator backbone), 2.73 - 4.02 (m, backbone repeating units). ¹³C NMR (700 MHz, D2O, δ (ppm)): 43.07 (s, 2^{nd} carbon next to amino group), 60.89 - 79.76 (m, polymer backbone).

### Synthesis of dPG maleimide

dPGNH2 (1.4 g, 0.14 mmol, 1 eq.), 6- maleimidohexanoic acid (0.15 g, 0.7 mmol, 5 eq.), and *N*-hydroxysuccinimide (0.13 g, 1.12 mmol, 8 eq.) were added to DMF (20 mL). *N,N'-*Diisopropylcarbodiimide (DIC, 0.17 mL, 1.12 mmol, 8 eq.) was then added to the mixture and it was allowed to stir overnight at room temperature. The crude mixture was afterwards subjected to dialysis against water (MWCO = 2 kDa) for 2 days. The purified product was later collected and kept in an aqueous solution with 85% isolated yield. ¹H NMR (700 MHz, D2O, δ (ppm)): 0.92 (3H, broad s, initiator backbone), 1.32 (2H, broad s), 1.61 (4H, broad s), 2.28 (2H, broad s), 3.26 - 4.04 (m, backbone repeating units), 6.89 (2H, broad s).

### Synthesis of dPGSNH₂

An initial mixture was made by the addition of dPG (6 g, 0.6 mmol, 1 eq.) and TEA (0.83 mL, 6 mmol, 10 eq.) were added to DMF (60 mL). The mixture was then cooled down with the help of an ice bath. Subsequently methanesulfonyl chloride (0.37 mL, 4.8 mmol, 8 eq.) was added dropwise to the stirring solution, which was then allowed to stir overnight. Following the addition of NaN₃ (0.62 g, 9.6 mmol, 16 eq.), the reaction mixture was allowed to stir as it was heated to 60 °C. After 2 days, sulfur trioxide pyridine complex (23.87 g, 150 mmol, 250 eq.) was added to the mixture and then allowed to run for 2 days at room temperature. The crude mixture was purified by first neutralizing the solution with NaOH and then dialyzing it first against brine, followed by water for 2 more days. Dialysis tubes with a 2 kDa molecular weight cut-off were used for both these cases. After purification, TCEP (1.38 g, 4.8 mmol, 8 eq.) was added to the aqueous solution and it was allowed to stir for 3 days. The crude mixture was then purified by dialysis against water (MWCO = 2 kDa) carried out for 2 d. Finally, the aqueous solution of the product was lyophilized overnight and collected as a solid pale yellow powder with 65% isolated yield with 4 % as degree of sulfation. ¹H NMR (600 MHz, D2O, δ (ppm)): 0.93 (3H, broad s, initiator backbone), 3.45 - 4.74 (m, backbone repeating units), Elemental analysis: N 0.84, C 20.52, S 14.94, H 3.71.

### Functionalization of dPGS maleimide

6-maleimidohexanoic acid (0.07 g, 0.35 mmol, 7 eq.), and *N*-hydroxysuccinimide (0.05 g, 0.45 mmol, 9 eq.) were added to a solution of DMF (5 mL). This was followed by the addition of DIC (0.07 mL, 0.45 mmol, 9 eq.) and the mixture was stirred. After 30 minutes, the aqueous solution (5 mL) of dPGSNH2 (1 g, 0.05 mmol, 1 eq.) was added to the reaction mixture and then allowed to stir overnight at room temperature. The crude product was purified by dialyzing the mixture against water with for 2 days (MWCO = 2 kDa). Finally, the aqueous solution was lyophilized and the final product was obtained as a solid, pale yellow powder in 87% isolated yield. ¹H NMR (700 MHz, D2O, δ (ppm)): 0.94 (3H, broad s, initiator backbone), 1.30 (2H, broad s), 1.62 (4H, broad s), 2.26 - 2.29 (2H, broad s), 2.48 - 2.52 (2H, broad s), 3.40 - 4.75 (m, backbone repeating units), 6.93 (2H, broad s). Elemental analysis: N 1.95, C 21.75, S 16.23, H 3.62

### Cytotoxicity studies

All cell experiments were conducted according to German genetic engineering laws and German biosafety guidelines in the laboratory (safety level 1). A549, HBE and Vero E6 cells were seeded in a 96-well plate at a density of 5 × 10⁴ cells/mL in 90µl DMEM medium per well over night at 37°C and 5% CO₂. 10µl of sample (solved in deionized water) were added in serial dilutions including positive (1% SDS) and negative controls (medium, H₂O) and incubated for another 24 h at 37_{°}C and 5% CO₂. For background subtraction, also wells containing no cells but only sample were used. After 24h incubation, the CCK8 solution was added (10 µl/well) and absorbance (450 nm/650 nm) was measured after approximately 3h incubation of the dye using a Tecan plate reader (Infinite pro200, TECAN-reader Tecan Group Ltd.) Measurements were performed in triplicates and repeated three times. The cell viability was calculated by setting the non-treated control to 100% and the non-cell control to 0% after subtracting the background signal using the Excel software.

### Virus binding assay

The samples were disinfected by UV-irradiation first for 30 min. Then they were incubated with HSV-1-GFP solution (300 µL, -20,000 PFU/mL) for 1 hour with constant shaking. Afterwards, the virus particles in the solution was titrated by a plaque assay on theVeroE6 line with DMEM (0.9% methycellulose) as overlay medium. The plaques were counted after 2 days with a fluorescence microscope (Axio, Zeiss, Germany).

### List of references cited in the preceding sections

[1] P. G. Arduino, S. R. Porter, J Oral Pathol Med 2008, 37, 107.
[2] R. F. Laine, A. Albecka, S. van de Linde, E. J. Rees, C. M. Crump, C. F. Kaminski, Nature Communications 2015, 6, 5980.
[3] C. Dogrammatzis, H. Waisner, M. Kalamvoki, Viruses 2021, 13, 17.
[4] S. Crimi, L. Fiorillo, A. Bianchi, C. D'Amico, G. Amoroso, F. Gorassini, R. Mastroieni, S. Marino, C. Scoglio, F. Catalano, P. Campagna, S. Bocchieri, R. De Stefano, M. T. Fiorillo, M. Cicciù, Viruses 2019, 11*.*
[5] B. Ziem, W. Azab, M. F. Gholami, J. P. Rabe, N. Osterrieder, R. Haag, Nanoscale 2017, 9, 3774.
[6] P. Dey, T. Bergmann, J. L. Cuellar-Camacho, S. Ehrmann, M. S. Chowdhury, M. Zhang, I. Dahmani, R. Haag, W. Azab, ACS Nano 2018, 12, 6429.
[7] P. Pouyan, C. Nie, S. Bhatia, S. Wedepohl, K. Achazi, N. Osterrieder, R. Haag, Biomacromolecules 2021, 22, 1545.
[8] H. Kamata, X. Li, U.-i. Chung, T. Sakai, Advanced Healthcare Materials 2015, 4, 2360.
[9] A. Herrmann, R. Haag, U. Schedler, Adv Healthc Mater 2021, 10, e2100062.
[10] B. Gyarmati, B. Vajna, Á. Nemethy, K. László, A. Szilágyi, Macromolecular Bioscience 2013, 13, 633.
[11] T. J. Sanborn, P. B. Messersmith, A. E. Barron, Biomaterials 2002, 23, 2703.
[12] Y. Zhang, Q. Yao, C. Xia, X. Jiang, P. G. Wang, ChemMedChem 2006, 1, 1361.
[13] R. Bansil, B. S. Turner, Curr Opin Colloid In 2006, 11, 164.
[14] N. Tsurutaro, Bulletin of the Chemical Society of Japan 1952, 25, 88.
[15] S. K. Lai, Y. Y. Wang, D. Wirtz, J. Hanes, Adv Drug Deliv Rev 2009, 61, 86.
[16] J. Li, D. J. Mooney, Nature Reviews Materials 2016, 1, 16071.
[17] A. Sharma, B. Thongrom, S. Bhatia, B. von Lospichl, A. Addante, S. Y. Graeber, D. Lauster, M. A. Mall, M. Gradzielski, R. Haag, Macromolecular Rapid Communications 2021, 42, 2100303.
[18] B. von Lospichl, S. Hemmati-Sadeghi, P. Dey, T. Dehne, R. Haag, M. Sittinger, J. Ringe, M. Gradzielski, Colloids Surf B Biointerfaces 2017, 159, 477.
[19] Y. Tsuji, X. Li, M. Shibayama, Gels (Basel, Switzerland) 2018, 4, 50.
[20] K. S. Anseth, C. N. Bowman, L. Brannon-Peppas, Biomaterials 1996, 17, 1647.
[21] S. S. Olmsted, J. L. Padgett, A. I. Yudin, K. J. Whaley, T. R. Moench, R. A. Cone, Biophys J 2001, 81, 1930.
[22] H. Frey, R. Haag, J Biotechnol 2002, 90, 257.
[23] A. Sunder, R. Hanselmann, H. Frey, R. Mülhaupt, Macromolecules 1999, 32, 4240.
[24] R. Haag, A. Sunder, J.-F. Stumbe, Journal of the American Chemical Society 2000, 122, 2954.
[25] M. Wallert, J. Plaschke, M. Dimde, V. Ahmadi, S. Block, R. Haag, Macromolecular Materials and Engineering 2021, 306, 2000688.

## Claims

1. Hydrogel, comprising dendritic polyglycerol units and polyether units, **characterized in that** the dendritic polyglycerol units correspond to general formula (I) and the polyether units correspond to general formula (II), wherein the dendritic polyglycerol units and the polyether units are covalently bound to each other via the bonds illustrated by dashed lines in formula (I) and formula (II): 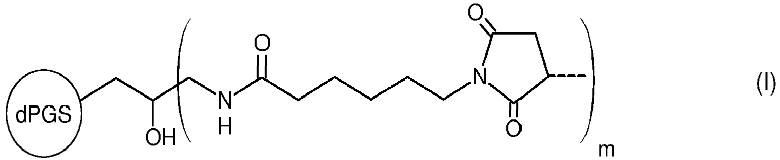 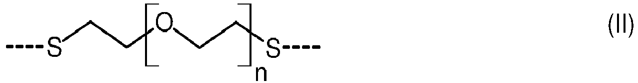 wherein
dPGS is a dendritic polyglycerol core comprising terminal hydroxyl groups and terminal sulfate or sulfonate substituents, wherein a degree of substitution of the dendritic polyglycerol core with sulfate or sulfonate substituents lies in a range from 10 % to 98 %,
m is 2 % to 10 % of all terminal groups of the dendritic polyglycerol core that are not substituted with the sulfate or sulfonate substituents,
n is a number lying in range from 1 to 1000.

2. Hydrogel according to claim 1, **characterized in that** the degree of substitution of the dendritic polyglycerol core lies in a range from 70 % to 98 %.

3. Hydrogel according to claim 1 or 2, **characterized in that** each dendritic polyglycerol core carries 4 to 13 maleimide substituents corresponding to general formula (III):

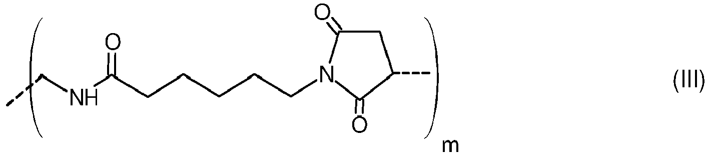

4. Hydrogel according to any of the preceding claims, **characterized in that** the hydrogel has a gel concentration lying in a range of from 3 % (w/v) to 10 % (w/v).

5. Hydrogel according to any of the preceding claims, **characterized in that** the hydrogel has a mesh size, measured at 37 _{°}C in a non-swollen state of the hydrogel, lying in a range of from 10 nm to 100 nm.

6. Use of a hydrogel according to any of the preceding claims for binding viruses in vitro.

7. Use according to claim 6, **characterized in that** the viruses are influenza viruses, immunodeficiency virus type-1 (HIV), dengue viruses, coronaviruses, or herpes simplex viruses.

8. Use of a hydrogel according to any of claims 1 to 5 as matrix for cultivating and/or expanding cells and/or organoids in vitro.

9. Hydrogel according to any of claims 1 to 5 for use in antiviral therapy of humans or animals.

10. Hydrogel according to any of claims 1 to 5 for use as mucus replacement, for use as cartilage replacement, or for use as synovial fluid replacement in a patient in need thereof.

11. Pharmaceutical composition comprising a hydrogel according to any of claims 1 to 5.

12. Method for manufacturing a hydrogel according to any of claims 1 to 5, **characterized by** the following step: mixing an aqueous solution of a dendritic polyglycerol maleimide corresponding to general formula (IV) with an aqueous solution of a poly(ethylene glycol) dithiol corresponding to general formula (V): 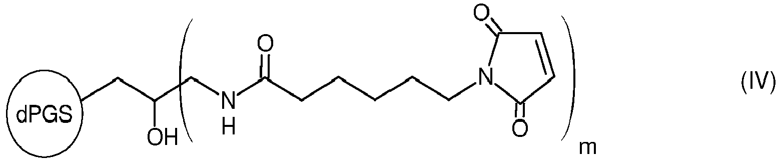 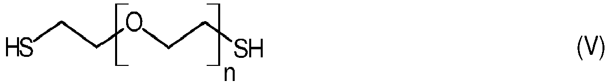 **wherein**
dPGS is a dendritic polyglycerol core comprising terminal hydroxyl groups and terminal sulfate or sulfonate substituents, wherein a degree of substitution of the dendritic polyglycerol core with sulfate or sulfonate substituents lies in a range from 10 % to 98 %,
m is 2 % to 10 % of all terminal groups of the dendritic polyglycerol core that are not substituted with the sulfate or sulfonate substituents,
n is a number lying in range from 1 to 1000.

13. Method according to claim 12, **characterized in that** a molar ratio between the poly(ethylene glycol) dithiol and the dendritic polyglycerol maleimide lies in a range from 1:1.5 to 9:1.

14. Method according to claim 12 or 13, **characterized in that** the dendritic polyglycerol maleimide is manufactured by reacting a dendritic polyglycerol amine having a dendritic polyglycerol core comprising terminal hydroxyl groups and terminal sulfate or sulfonate substituents, wherein a degree of substitution of the dendritic polyglycerol core with sulfate or sulfonate substituents lies in a range from 10 % to 98 %, with 6-maleimidohexanoic acid, N-hydroxysuccinimide and N,N'-diisopropylcarbodiimide.

15. Method according to claim 14 in that the dendritic polyglycerol amine carries sulfate substituents and is manufactured by mesylating a non-substituted dendritic polyglycerol to obtain a mesylated dendritic polyglycerol, adding an azide to the mesylated dendritic polyglycerol to form a dendritic polyglycerol azide, and reducing and sulfating the dendritic polyglycerol azide with triphenylphosphine and sulfur trioxide to obtain the dendritic polyglycerol amine carrying sulfate substituents.
